# EUROPEAN PATENT SPECIFICATION

(11) **EP 1 810 025 B1**
(45) Date of publication and mention of the grant of the patent: **14.04.2010**
(21) Application number: 05796587.3
(22) Date of filing: 20.10.2005
(51) Int. Cl.: G01N 33/50, G01N 15/12

(54) **LYSING REAGENT FOR SIMULTANEOUS ENUMERATION OF DIFFERENT TYPES OF BLOOD CELLS IN A BLOOD SAMPLE**
LYSEREAGENS ZUR GLEICHZEITIGEN AUSZÄHLUNG UNTERSCHIEDLICHER ARTEN VON BLUTZELLEN IN EINER BLUTPROBE
REACTIF LYSOGENE PERMETTANT LE RECENSEMENT SIMULTANE DE DIFFERENTS TYPES DE CELLULES SANGUINES DANS UN ECHANTILLON SANGUIN

(30) Priority: 20.10.2004 DK 200401609
(43) Date of publication of application: 25.07.2007
(73) Proprietor: Chempaq A/S, 3520 Farum (DK)
(72) Inventor: EKBERG, Björn, SE-21774 Malmö (SE)
(74) Representative: Jakobsen, Gert Hoey
(86) International application number: PCT/DK2005/000681
(87) International publication number: WO 2006/042555

(56) References cited:
- WO-A-01/11338
- US-A- 4 286 963
- US-A- 4 745 071
- US-A- 4 962 038
- US-A- 5 227 304
- US-A- 5 958 781

## Description

### TECHNICAL FIELD

The present invention relates to a reagent for use in the simultaneous automatic electronic enumeration and volumetric discrimination, for example by Coulter counting, of different types of blood cells, such as leukocytes, thrombocytes, etc, in blood. Further, the present invention relates to a Coulter (impedance) counting apparatus containing the reagent, for example a Coulter counting apparatus with a disposable cartridge for characterizing cells suspended in a liquid, especially a self-contained disposable cartridge for single-use analysis, such as for single-use analysis of a small quantity of whole blood.

### BACKGROUND OF THE INVENTION

Information on the content of leukocytes (white blood cells), their subpopulations and thrombocytes (platelets) is an important tool for the physician in order to diagnose different diseases and monitor treatment. Furthermore, the concentration of hemoglobin, directly related to the number of erythrocytes, in the blood sample is also of great importance.

Thus, much effort has over the years been devoted to the development of automated blood cell counting systems. Automated blood cell counting systems can be divided into two major groups: those relying on the impedance cell sizing principle (equal to the Coulter principle) and those relying on the flow cytometry principle. Examples of such automated systems are Coulter AcT Diff based on impedance cell sizing and Bayer ADVIA 120 based on flow cytometry. Both principles can be combined with spectrophotometric techniques for analysis of additional components in the blood, such as hemoglobin.

US 4,962,038 disclose a reagent system containing a blood diluent and a lysing agent for routine enumeration of traditional hemogram values and the determination of leukocytes. The blood diluent comprises procaine hydrochloride, ADA, chlorhexidine diacetate, 1-hydroxypyridine-2-thione, and dimethylolurea, and the lysing agent comprises at least one quaternary ammonium salt and an alkali metal cyanide.

US 4,745,071 disclose a diluent agent, a lysing agent, and a detergent for differentiation between and enumeration of lymphocytes, neutrophils and leukocytes.

The blood diluent comprises 1,3-dimethylurea, 1-hydroxypyridine-2-thione, and ADA, the lysing agent comprises a single quaternary ammonium salt and potassium cyanide, and the detergent comprises, in addition to the components of the diluent, a wetting agent diazopon.

US 5,227,304 disclose a diluent solution in combination with a detergent solution for routine enumeration of blood cells, a blood sample is divided into a red cell aliquot for enumeration of red blood cells (erythrocytes) together with platelets (thrombocytes), and a white cell aliquot for the enumeration of white blood cells (leukocytes) and hemoglobin. In addition to the described solutions a lysing solution (as decribed in US 4 745 071, see above) is needed for enumeration of the white cell aliquot. The diluent solution comprises imidazole, dimethylurea, EDTA, sodium omadine, triadine-10, and triadine-3, and the detergent solution comprises triadine-10, sodum omadine, and Brij 35.

US 5,958,781 discloses a method for determining hemoglobin and leukocytes with a diluent comprising EDTA, imidazole, and a mixture of hexahydro-1,3,5-tris(2-hydroxyethyl)-s-triazine and sodium 2-pyridinethiol-1-oxide and a lysing reagent comprising at least one quaternary ammonium salt and a hydroxylamine salt.

US 4,286,963 discloses a lytic diluent and a method for rapid lysing of red blood cells in whole blood for a differential determination of lymphoid and myeloid populations of leukocytes, and measurement of hemoglobin, particularly for use in automatic particle counting systems. The diluent is a mixture of at least one quaternary ammonium salt having surface active properties, and an additive in suitable proportions, buffered to an acid pH of 3.5 to 5.0. The additive includes 2-phenoxyethanol and other short chain alkanols substituted by one phenyl or phenoxy, as well as certain polyhydroxy compounds.

Traditionally, erythrocytes and thrombocytes are quantified in a blood sample after addition of a diluent solution, whereas a lysing agent is added to the same or a separate blood sample to release hemoglobin from the erythrocytes and permit the quantification of the leucocytes together with an analysis of the hemoglobin. A full analysis of both erythrocytes, thrombocytes, leucocytes and hemoglobin, thus requires several steps.

Accordingly, there is a need for developing a reagent that combines the function of a diluent and a lysing agent, and which simultaneously allows a manual or an automated enumeration of various blood cells such as, e.g., thrombocytes and leukocytes, together with an analysis of hemoglobin.

A simultaneously identification and quantification of leukocytes and thrombocytes using one combined reagent only, would require that a lysing agent can lyse the erythrocytes without significantly affecting the thrombocytes. Typically, known lysing agents severely decreases the size of the thrombocytes, whereby, in connection with quantification, signals generated by the remains of the thrombocytes overlap with the particle background noise level. Furthermore, it is believed that the background noise is to a large extent generated by debris stemming from erythrocyte membranes. The further the background noise level is reduced the more accurate the thrombocyte particles can be identified and quantified.

Known lysing agents typically contain a cyanide salt and due to the toxicity of hydrogen cyanide such agents must have an alkaline pH. At a pH above 8, the size of e.g. thrombocytes changes drastically. In order to obtain an accurate and precise simultaneously analysis of all the above mentioned blood cells, together with an analysis of hemoglobin, the pH-value of a combined reagent system must be close to neutral, i.e., well below 8. It has previously been described that a medium used for enumeration of thrombocytes must, in addition to having a suitable osmolality, have a pH in the range of 6.5 - 7.6 (US 5,935,857, US 4,745,071, US 4,185,964) to preserve the cell-volume of the thrombocytes.

### SUMMARY OF THE INVENTION

The present invention relates to a reagent comprising a hemoglobin-stabilizing/methemoglobin ligand compound selected from the group consisting of imidazole, imidazole derivatives, and combinations thereof, at least two quaternary ammonium salts, 1,3-dimethylurea and/or salts thereof; and an organic buffer. The reagent is useful as a combined diluent and lysing agent allowing a simultaneously counting or analysis of various blood cells as well as analysis of hemoglobin.

Accordingly, the present invention provides a method for determining the content of blood cells in a blood sample comprising the steps of: mixing a blood sample with a reagent according to the invention; and analyzing the content of the blood sample by employment of the Coulter principle.

Further aspects of the invention, will become apparent from the following description.

### DETAILED DESCRIPTION OF THE INVENTION

It is an object of the present invention to provide a reagent that combines the function of a diluent and a lysing agent, and which simultaneously allows a manual or an automated counting or analysis of various blood cells and analysis of hemoglobin. Hereby enabling the integration of dilution and lysing of a blood sample in one single step.

It is a further object of the present invention to provide a reagent that preserves the volume of the thrombocytes and improves the decomposition of unwanted cell debris from red blood cells (erythrocytes), thereby reducing background noise and facilitating an improved and more precise analysis or counting of thrombocytes simultaneously with other blood cells.

The reagent according to the present invention contains an aqueous medium. The inventors has surprisingly found, that the above-mentioned and other objects are fulfilled by a reagent comprising, preferably in addition to other compounds mentioned below, a hemoglobin-stabilizing/methemoglobin ligand compound selected from the group consisting of imidazole, imidazole derivatives, and combinations thereof, at least two quaternary ammonium salts, 1,3-dimethylurea and/or salts thereof; and an organic buffer.

It is an important advantage of the present invention that while the reagent affects various types of blood cells, e.g. the erythrocytes are substantially eliminated, the leukocytes decrease in volume and the thrombocytes also decrease in volume, the composition of the lysing reagent according to the invention is accurately controlled so that the decrease in volume of the thrombocytes is not significant for the ability of the thrombocytes to be counted in a Coulter counter. It has been verified that the identified parts of the remains of the thrombocytes were related to the original content of thrombocytes in the blood samples according to comparative methods. This is further described in example 1 below.

In order to correctly and easily quantify the content of hemoglobin in blood samples, this analyte has to be released from the erythrocytes (lysis) and preferably converted to a suitable complex. The complex has to be suitable from a spectrophotometric point-of-view. The major hemoglobin species present in the blood are transformed to the corresponding complexed methemoglobin in two steps. The first oxidizing step is achieved in the present invention by oxidation of deoxy-hemoglobin and oxy-hemoglobin by the action of the quaternary ammonium salts and oxygen. The oxidized hemoglobin species, i.e. methemoglobin, is in the second step according to the invention complexed with imidazole resulting in a methemoglobin-imidazole complex, which is stable and has well-defined spectrophotometric properties. The complex may thus be spectrophotometrically quantified using specific wavelengths, e.g., 545 and 880 nm. The former wavelength quantifying the complex and the latter one compensating for turbidity in the blood sample and other absorbances, which are not attributed to the content of hemoglobin.

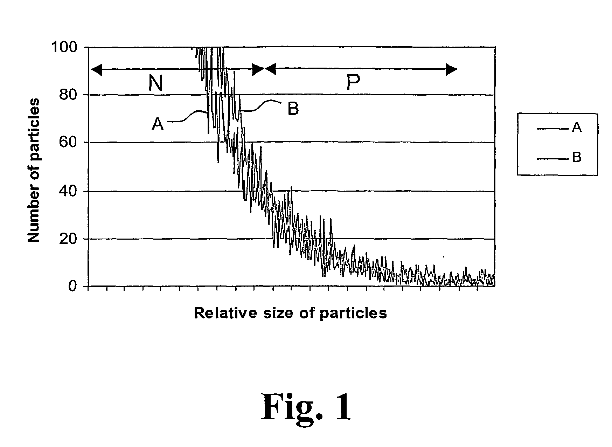

Thus, the action of the quaternary ammonium salts is two-fold: lysing the erythrocytes and enabling the oxidation of hemoglobin. A draw back of using quaternary ammonium salts are their simultaneous lysing action on leukocytes and thrombocytes. This action is compensated by the presence of 1,3-dimethylurea in the present invention. The 1,3-dimethylurea helps the preservation of, in particular, the leukocytes and thrombocytes and the identification of the three subcell types lymphocytes, monocytes and granulocytes. Accordingly, the reagent according to the present invention improves preservation of the volume of thrombocytes thus improving ability to count thrombocytes, e.g. by lysing the thrombocytes to a smaller degree (cf. Fig. 1).

The erythrocyte cell membrane disintegrates completely during the action of the lysing reagent. Since the erythrocytes do not contain any cell organelles, small cell membrane debris is the only remains of this blood cell type. This debris contributes significantly to the generation of the background-noise of automatic blood cell counting systems based on impedance cell sizing.

It is an important advantage of the present invention that the reagent improves the decomposition of unwanted cell debris from red blood cells (erythrocytes), thereby reducing background noise and facilitating an improved and more precise analysis or counting of thrombocytes.

The hemoglobin stabilizing compound in the reagent is selected from the group consisting of imidazole, imidazole derivatives, and combinations thereof and may be in a concentration in the range from 10 mmol/L to 100 mmol/L, such as from 25 mmol/L to 75 mmol/L, from 40 mmol/L to 60 mmol/L, and preferably about 50 mmol/L. The hemoglobin stabilizing compound is preferably imidazole in a concentration of 50 mmol/L.

The blood cells in a blood sample are lysed by at least two quaternary ammonium salts. The quaternary ammonium salts suitable for use in the present invention has the formula: 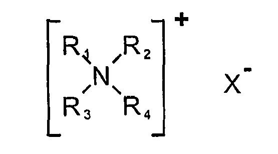 where R₁ is a long chain alkyl, alkenyl, or alkynyl radical having 10 to 18 carbon atoms, R₂, R₃, and R₄ are short chain alkyl, alkenyl or alkynyl radicals having 1 to 6 carbon atoms or just hydrogen, and X⁻ is a salt forming ion such as Cl⁻, Br⁻, I⁻, PO₄³⁻, CH₃SO₄⁻.

A combination of dodecyltrimethylammonium chloride and hexadecyltrimethylammonium bromide is preferred, but other quaternary ammonium salts may be for example tetradecyltrimethylammonium bromide or hexadecyldimethylethylammonium bromide.

The total concentration of the at least two quaternary ammonium salts in the reagent may be in the range from 1.0 mmol/L to 10 mmol/L, such as from 3.0 mmol/L to 9.0 mmol/L, from 4.5 mmol/L to 8.5 mmol/L, and preferably 6.5 mmol/L.

In a preferred embodiment of the invention the at least two quaternary ammonium salts is a combination of dodecyltrimethylammonium chloride and hexadecyltrimethylammonium bromide in concentrations of 5.6 mmol/L and 0.9 mmol/L, respectively

As described above the 1,3-dimethylurea acts substantially as a cell stabilizing and antibacterial agent. The concentration of 1,3-dimethylurea and/or salts thereof in the reagent according to the present invention may be in the range from 10 mmol/L to 25 mmol/L, such as from 12 mmol/L to 23 mmol/L, from 14 mmol/L to 20 mmol/L, from 16 mmol/L to 18 mmol/L /L, and preferably about 17 mmol/L. In a preferred embodiment of the invention 1,3-dimethylurea is in a concentration of 17 mmol/L.

The reagent according to the present invention further comprises an organic buffer, this organic buffer is a compound such as ADA (N-(2-acetamido)iminodiacetic acid), HEPES (2-(4(2-hydroxyethyl)-1-piperazine)ethanesulphonic acid), MOPS (3-(N-morpholino)propane sulphonic acid and PIPES (piperazine-N,N'-bis(2-ethane sulphonic acid) or combinations thereof and/or other organic buffers. Suitable concentrations of ADA, HEPES, MOPS and PIPES or combinations thereof in the reagent may be in the range from 2 mmol/L to 8 mmol/L, such as from 4 mmol/L to 6 mmol/L, from 5 mmol/L to 5.6 mmol/L, and preferably 5.3 mmol/L. In a preferred embodiment of the invention the organic buffer is ADA with a concentration in the range from 2 mmol/L to 8 mmol/L, such as preferably from 4 mmol/L to 6 mmol/L, more preferably from 5 mmol/L to 5.6 mmol/L, and even more preferably 5.3 mmol/L.

In addition to its buffering effect, ADA contributes to minimizing bacterial growth by its metal-ion chelating properties. Finally, ADA further assists the quaternary ammonium salts in reducing the red blood cells debris to a size minimizing interference with the quantification of thrombocyte particles close to the background noise as described above.

As mentioned above, there is a need to compensate for the action of the quaternary ammonium salts on the thrombocytes and leukocytes. In summary, the presence of 1,3-dimethylurea contributes to the preservation of the thrombocytes and the leukocyte subpopulations.

In a preferred embodiment, the reagent according to the present invention may further comprise procaine, procaine hydrochloride or other salts thereof. Procaine hydrochloride acts substantially as a cell stabilizing agent, and the concentration of procaine hydrochloride and/or salts thereof in the reagent may be in the range from 0.2 mmol/L to 1.6 mmol/L, such as from 0.4 mmol/L to 1.4 mmol/L, from 0.6 mmol/L to 1.2 mmol/L, from 0.8 mmol/L to 1.0 mmol/L, and preferably 0.9 mmol/L.

Furthermore, to obtain a correct enumeration of the thrombocytes the cell volume must be preserved. At an elevated pH-value the size of the remains of the thrombocytes decrease further, whereby the thrombocytes generates signals that partly overlap the background noise signals, making the quantification of them more inaccurate. The pH of the reagent according to the present invention may therefore be adjusted to a suitable value in the range from 6 to 8, such as preferably in a range from 6.5 to 7.5, and more preferably about 7.1 with sulfuric acid or other suitable acids, such as hydrochloric acid, and phosphoric acid. A person skilled in the art will recognize that when a specific combination of substances in the reagent gives an pH-value below the desired pH-value, then an alkaline substance such as an inorganic base e.g. sodium hydroxide, may instead be used to adjust the pH-value. The osmolality may be adjusted to the range from 200 mosmol/L to 400 mosmol/L, such as preferably from 250 mosmol/L to 380 mosmol/L, more preferably from 300 mosmol/L to 350 mosmol/L, and even more preferably 330 mosmol/L with an inorganic salt, such as sodium chloride, and/or sodium sulphate. In a preferred embodiment of the invention, the osmolality of the reagent is adjusted with sodium chloride and sodium sulphate. The blood sample may contain EDTA (ethylenediaminetetraacetic acid), salts thereof or any other suitable anticoagulating compound such as heparin to prevent the blood sample from clotting.

In a specific embodiment of the invention, the reagent according to the invention comprises imidazole in a concentration of 50 mmol/L; dodecyltrimethylammonium chloride in a concentration of 5.6 mmol/L; hexadecyltrimethylammonium bromide in a concentration of 0.9 mmol/L; 1,3-dimethylurea in a concentration of 17 mmol/L; ADA (N-2-(acetamido)iminodiacetic acid) in a concentration of 5.3 mmol/L; and de-ionized water; wherein pH is adjusted to 7.1 with diluted sulfuric acid and the osmolality is adjusted with sodium chloride and sodium sulphate.

In another specific embodiment of the invention, the reagent according to the invention comprises imidazole in a concentration of 50 mmol/L; dodecyltrimethylammonium chloride in a concentration of 5.6 mmol/L; hexadecyltrimethylammonium bromide in a concentration of 0.9 mmol/L; 1,3-dimethylurea in a concentration of 17 mmol/L; ADA (N-2-(acetamido)iminodiacetic acid) in a concentration of 5.3 mmol/L; procaine hydrochloride in a concentration of 0.9 mmol/L; and de-ionized water; wherein pH is adjusted to 7.1 with diluted sulfuric acid and the osmolality is adjusted with sodium chloride and sodium sulphate.

Another aspect of the present invention is with regard to a method of determining the content of blood cells such as, e.g., thrombocytes, leucocytes, subpopulations of leucocytes, i.e. lymphocytes, monocytes, granulocytes, and hemoglobin, in a blood sample, e.g. an ex-vivo blood sample, the method comprising the steps of:
i) Mixing a blood sample, e.g. an ex-vivo blood sample, with a reagent according to the invention, and
ii) Analyzing the content of the blood sample, e.g., enumerating leukocytes, lymphocytes, monocytes, granulocytes and thrombocytes, by employment of the Coulter principle.

In a particular embodiment, the invention relates to a method of determining the content of thrombocytes, leukocytes and/or one or more leukocyte subpopulations such as, e.g., lymphocytes, monocytes and/or granulocytes. It is important to note that analysis of the various types of cells can be made simultaneously. The method according to the invention may furthermore comprise a step iii) Analyzing the content of the blood sample by spectrophotometric quantification of the hemoglobin content.

Furthermore, in the method according to the invention the ratio by volume between blood and reagent may be in the range from 1:10,000 to 1:200, such as from 1:1,000 to 1:300, from 1:600 to 1:400, from 1:550 to 1:450, and preferably 1:500. The selected ratio depends on the concentrations of the compounds in the reagent, concentrations of blood cells and desired performance characteristics of the analysis.

For example, the reagent according to the present invention may be contained in a cartridge utilized in an apparatus for enumeration of cells in a blood sample, e.g. an ex-vivo blood sample, the cartridge comprising a housing with a mixing chamber and a collection chamber separated by a wall containing an orifice for passage of the cells between the mixing chamber and the collection chamber. Cell characterization means are provided for characterizing cells passing through the orifice. The apparatus comprises a docking station for removably receiving the cartridge, the docking station comprising connectors, e.g. electrical and/or fluid connectors, for operational connection with the cell characterization means when the cartridge is received in the docking station.

In a preferred embodiment of the invention, step i) of the method according to the invention, is performed in a cartridge suitable for use in an apparatus for enumeration of cells in a blood sample, in which cartridge the reagent is present in a holding chamber that is connected with a mixing chamber in which the reagent is mixed with the blood sample; and step ii) is performed by subsequent passage of the blood sample comprising cells through an orifice positioned between the mixing chamber and a collection chamber, whereby the content of the blood is analyzed by employment of the Coulter principle by cell characterization means.

Additionally, in a preferred embodiment, step iii) of the method according to the invention, is performed in a volume metering chamber connected to the collection chamber and adapted to spectrophotometric quantification.

In addition the reagent according to the present invention may be employed in a conventional apparatus for counting and determination of content of blood cells and hemoglobin in blood samples, e.g. ex-vivo blood samples.

The reagent may further be used as a coating to enhance the capillary effect of a tube or tube-like device. A coating comprising the reagent according to the present invention has shown to enhance the capillary effect of a hydrophobic tube or tube-like device. The reagent is coated onto a surface by evaporation of the reagent on the surface to be coated. Accordingly, a further aspect of the present invention is a coating comprising a reagent according to the invention.

A further aspect of the invention is the use of a reagent as defined above for the determination of the content of one or more cell types in a blood sample. In a particular embodiment, the invention relates to the use for the determination of thrombocytes, leukocytes and/or one or more leukocyte subpopulations such as, e.g., lymphocytes, monocytes and/or granulocytes in the same blood sample. It is important to note that the determination of the various types of cell can be made simultaneously. As discussed above, a specific embodiment of the invention relates to the use of the reagent for the determination of all cell types and subpopulations mentioned above. Moreover, another advantage is that the same reagent is suitable for use in the determination of the hemoglobin content in the blood sample, i.e. by use of only one single reagent relevant information of the blood content is obtained.

In this context the term "simultaneous determination" refers to a simultaneous determination in one analysis in the one and same blood sample. Accordingly, a blood sample is mixed with a reagent according to the invention and the various blood cell populations can be determined simultaneously in one analysis.

### BRIEF DESCRIPTION OF THE DRAWINGS

Fig. 1 is a histogram comparing the reagent according to the present invention and another single reagent system regarding their ability to count thrombocytes.
Fig. 2 schematically illustrates a cartridge accommodating the reagent according to the present invention for analysis of a blood sample.
Fig. 3 shows an apparatus for analysis of blood sample content utilizing a cartridge accommodating the reagent according to the present invention.
Fig. 4 shows a favorable histogram for counting leukocytes and leukocyte subpopulations, obtained by using the reagent described in Table 2.
Fig. 5 shows a favorable histogram for counting thrombocytes obtained by using the reagent described in Table 4.
Fig. 6 shows a favorable histogram for counting leukocytes and the leukocyte subpopulations, obtained by using the reagent described in Table 4; the histogram is the same as in Fig. 5, but with a different Y-axis.

### EXAMPLES

The composition of the presently most preferred embodiment of the inventive reagent is listed in Table 1:

**Table 1**

| Compound | Concentration range |
|---|---|
| Imidazole | 48-52 mmol/L |
| Dodecyltrimethylammonium chloride | 5.4-5.8 mmol/L |
| Hexadecyltrimethylammonium bromide | 0.87-0.93 mmol/L |
| 1,3-dimethylurea | 16.5-17.5 mmol/L |
| Procaine hydrochloride | 0.87-0.93 mmol/L |
| ADA (N-2-(acetamido)iminodiacetic acid) | 5.0-5.6 mmol/L |
| Na₂SO₄ | 68-72 mmol/L |
| NaCl | 40-42 mmol/L |
| H₂SO₄, diluted | To obtain pH 7.1 |
| De-ionized water | To obtain 1 Liter |

| | |
|---|---|
| The pH of the reagent is adjusted to 7.1 using diluted sulphuric acid, H₂SO₄. | |

### EXAMPLE 1

### Correlation of test results using a reagent according to the invention with test results using a method of comparison

1 liter of reagent was prepared with a composition according to Table 2. The final osmolality of the prepared reagent was 330 mosmol/L.

**Table 2**

| Compound | Concentration |
|---|---|
| Imidazole | 50 mmol/L |
| Dodecyltrimethylammonium chloride | 5.6 mmol/L |
| Hexadecyltrimethylammonium bromide | 0.9 mmol/L |
| 1,3-dimethylurea | 17 mmol/L |
| Procaine hydrochloride | 0.9 mmol/L |
| ADA (N-2-(acetamido)iminodiacetic acid) | 5.3 mmol/L |
| Na₂SO₄ | 70 mmol/L |
| NaCl | 41 mmol/L |
| H₂SO₄ , diluted 1/9 (vol./vol.) with water. | 7 mL (to obtain pH 7.1) |
| De-ionized water | To obtain 1 liter |

Test results using a reagent according to the invention as described in table 2 were thereafter correlated with test results using a comparative system, the Beckman Coulter AcT Diff system. The test result includes quantification of leukocytes (including the three subpopulations lymphocytes, monocytes, and granulocytes), thrombocytes and hemoglobin. A total of 53 human whole blood samples were analyzed, where 3 µL of blood was mixed with 1.5 mL of reagent, which corresponds to a blood:reagent ratio by volume of 1:500. The tests were compared to tests performed on the Beckman Coulter AcT Diff system. The resulting correlations are listed in Table 3 for various concentration ranges of the content of leukocytes (including the three subpopulations lymphocytes, monocytes, and granulocytes), thrombocytes and hemoglobin in the blood samples. As can be seen from the table they demonstrate excellent correlation.

**Table 3**

| Analyte | Concentration range | Linear correlation factor |
|---|---|---|
| Leukocytes | (1.4 - 68)·10⁹/L | 1 |
| Lymphocytes* | (0.3 - 20)·10⁹/L | 0.99 |
| Monocytes* | (0.1 - 6.6)·10⁹/L | 0.96 |
| Granulocytes* | (1.0 - 31)·10⁹/L | 0.97 |
| Thrombocytes | (8 -1,170)·10⁹/L | 0.90 |
| Hemoglobin | (2.9 - 10.7) mmol/L | 0.97 |

| | | |
|---|---|---|
| * subpopulation of leukocytes | | |

Fig. 1 shows a histogram comparing number of particles counted in two samples of the same blood. The blood samples were prepared with a reagent according to the present invention and a known single reagent system (0.704 gram dodecyltrimethylammonium chloride, 0.150 gram hexadecyltrimethylammonium bromide and 1.796 gram of 1,2,4 - triazole dissolved in 360 ml of Diluide III Diff.((J.T. Baker prod.no. 3963)) and 116 ml of de-ionized water, respectively. The two prepared blood samples were analyzed using a cartridge as described below. Special electronic equipment was employed to record the cell size in a large number of intervals.

In Fig. 1 graph A shows the test result obtained with the blood sample with the known reagent, while B shows the test result obtained with the blood sample with the reagent according to the invention. P designates the thrombocytes size range, which ranges from about 1.5 µm to about 4 µm. N designates the background noise. It is noted that the counted number of thrombocytes using the reagent according to the present invention is larger than the counted number of thrombocytes using the known reagent, which is believed to be caused by a more gentle preparation of thrombocytes by the reagent according to the invention. Thus an improved preservation of thrombocytes, and thus a more precise and accurate analysis of the blood sample is obtained.

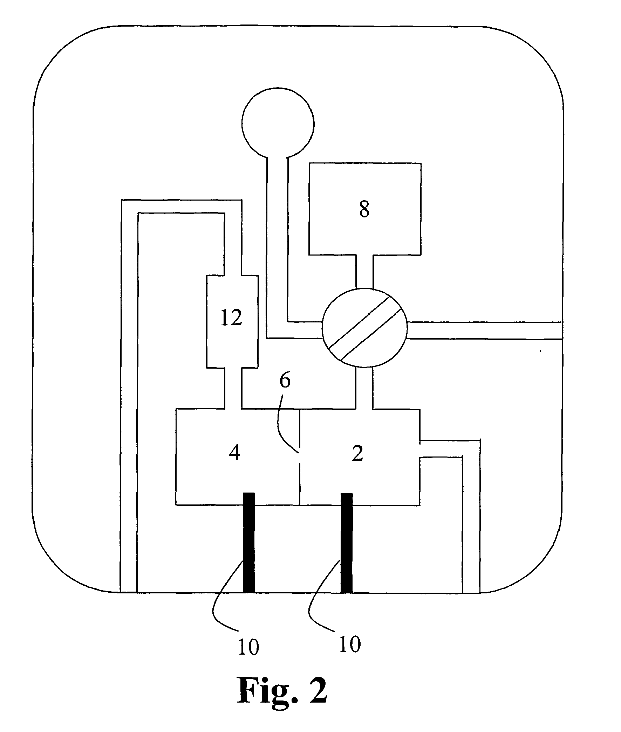

Fig. 2 schematically illustrates a disposable cartridge 1 holding a reagent according to the present invention. The cartridge is utilized in an apparatus for analysis of blood sample content that comprises a docking station and a reader. The cartridge comprises a housing with a mixing chamber 2 and a collection chamber 4 separated by a wall containing an orifice 6 for passage of the cells between the mixing chamber and the collection chamber. The reagent is accommodated in a holding chamber 8, and cell characterization means 10 are provided for characterizing cells passing through the orifice. Furthermore, the cartridge comprises a volume-metering chamber 12 connected to the collection chamber. A part of the cartridge, e.g. the mixing chamber 2, the collection chamber 4 and/or the volume-metering chamber 12, may be adapted for spectrophotometric quantification of hemoglobin content. In the shown embodiment, the volume-metering chamber 12 is adapted for spectrophotometric quantification of hemoglobin content in the blood.

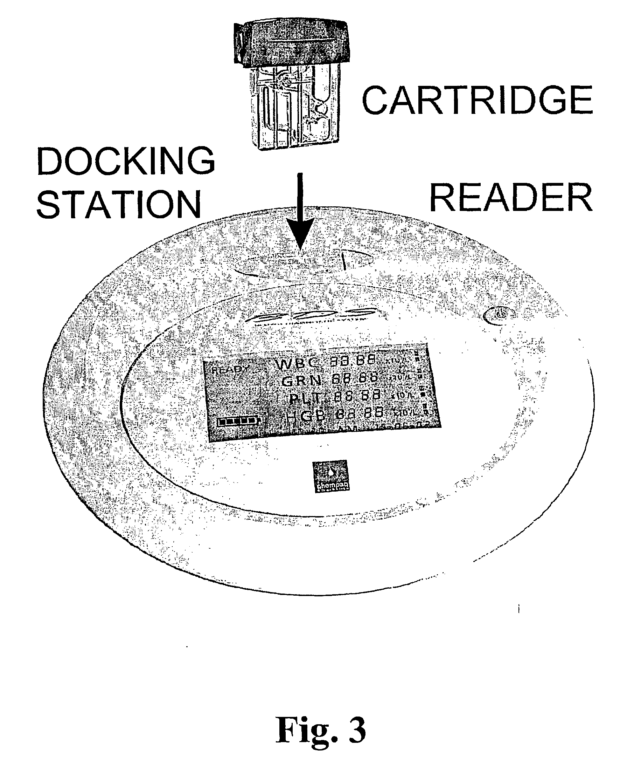

The apparatus for analysis of blood sample content is illustrated in Fig. 3 and comprises a docking station and a reader for removably receiving the cartridge, the docking station comprising connectors (not shown), e.g. electrical and/or fluid connectors, for operational connection with the cell characterization means when the cartridge is received in the docking station.

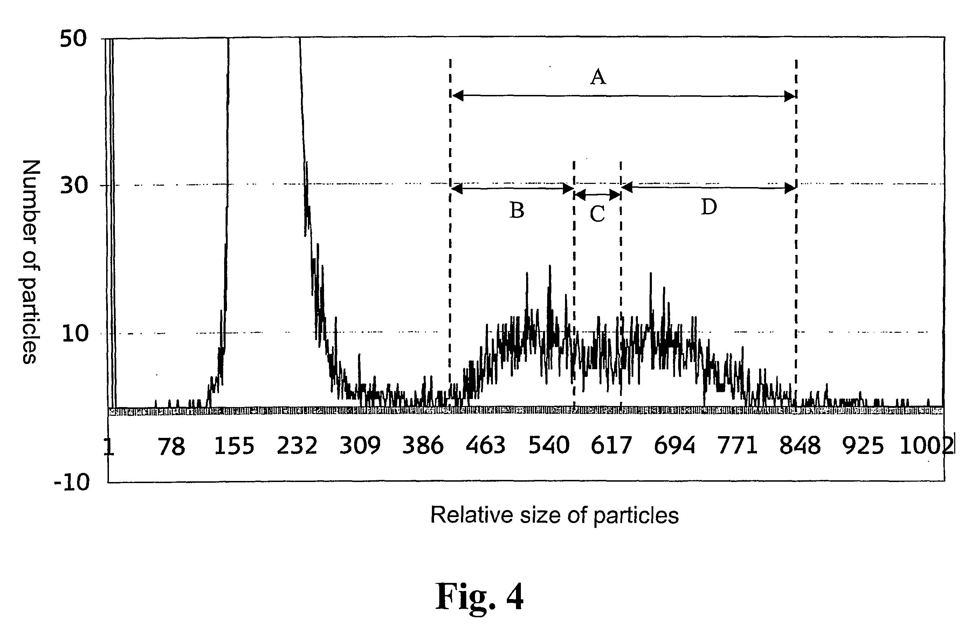

Fig. 4 shows a favorable histogram for counting thrombocytes, leukocytes and leukocyte subpopulations, obtained by using the reagent described in Table 2. A designates the part of the histogram that corresponds to leukocytes, B, C and D corresponds to lymphocytes, monocytes and granulocytes, respectively.

### EXAMPLE 2

### Reagent according to the invention

A reagent was prepared with a composition according to Table 4. The final osmolality of the prepared reagent was 330 mosmol/L.

**Table 4**

| Compound | Concentration |
|---|---|
| Imidazole | 50 mmol/L |
| Dodecyltrimethylammonium chloride | 5.6 mmol/L |
| Hexadecyltrimethylammonium bromide | 0.9 mmol/L |
| 1,3-dimethylurea | 17 mmol/L |
| ADA (N-2-(acetamido)iminodiacetic acid) | 5.3 mmol/L |
| Na₂SO₄ | 70 mmol/L |
| NaCl | 41 mmol/L |
| H₂SO₄, diluted 1/9 (vol./vol.) with water. | 7 mL (to obtain pH 7.1) |
| De-ionized water | To obtain 100 mL |

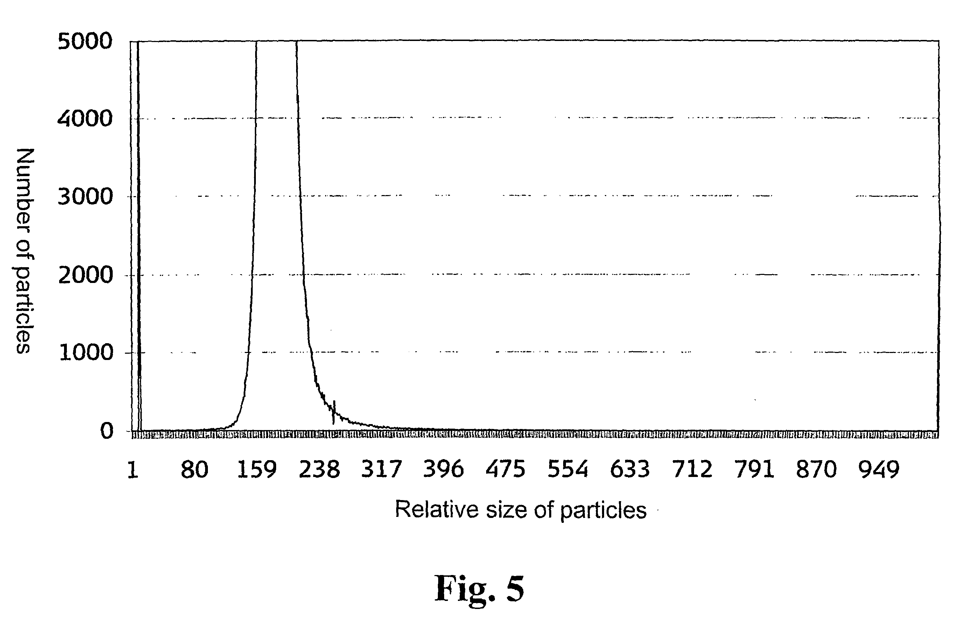

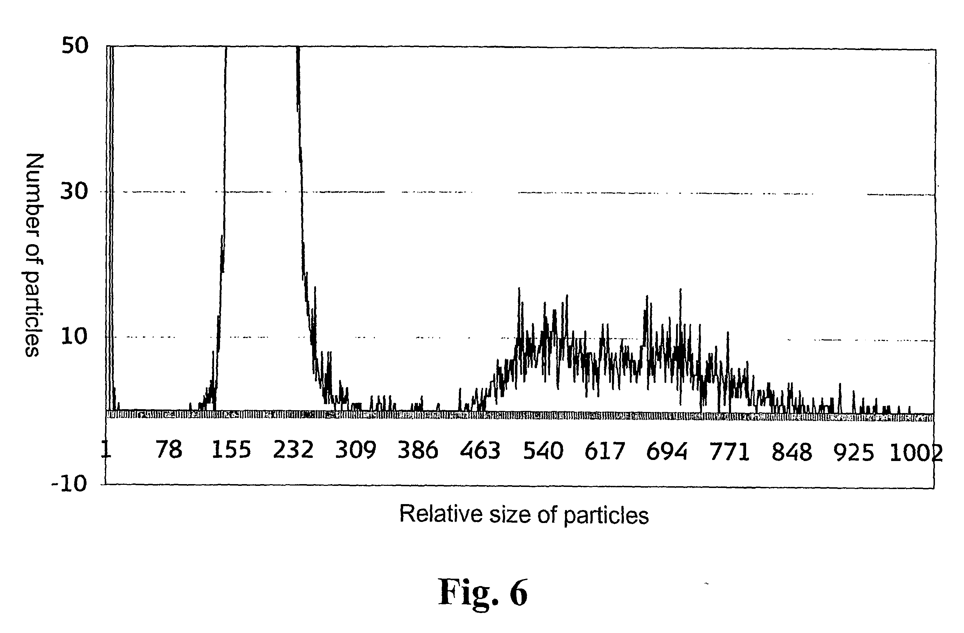

Test results obtained by using a reagent according to the invention, as described in table 4, to determine the content of the various blood cell can be seen in Fig. 5 showing a favorable histogram for counting thrombocytes, and in Fig. 6 showing a favorable histogram for counting leukocytes and leukocyte subpopulations. Fig. 5 and Fig. 6 are the one and same histogram, except that the Y-axis is different; to more clearly show the different cell-subtypes.

### EXAMPLE 3

### Enhancement of the capillary effect by use of the inventive reagent as a coating

Surprisingly, a coating comprising the reagent according to the present invention has shown to enhance the capillary effect of a hydrophobic tube or tube-like device. The reagent is coated onto a surface by evaporation of the reagent on the surface to be coated. The effect can be explained by taking the amphiphilic nature of the reagent components into consideration. The hydrophobic molecular parts of these adhere to the hydrophobic surface of the tube resulting in an orientation of the hydrophilic molecular parts of the reagent components out from the tube surface. The inlet of a hydrophilic sample, e.g. a blood sample, will subsequently be facilitated by interaction with the hydrophilic molecular parts of oriented reagent components. The enhancement of capillary effect, i.e. increased speed of inlet of e.g. a blood sample, is therefore mainly due to the hydrophilisation of hydrophobic surfaces in the capillary tube by the reagent.

The enhanced capillary effect using a reagent according to the present invention was observed by comparing the speed of inlet of blood samples in standard capillary glass tubes (microcaps). The capillary tubes were treated with a reagent according to example 1 and comparison were made with similar treatment using a solution of 3 % (weight/volume) of Brij 35 in de-ionised water - a well-known very efficient compound for enhancing hydrophilicity.
A) 10 glass microcaps with a volume of 20 µL, length 64 mm, (Drummond Inc. prod.no 1-000-0200) were filled with a reagent (as described in Example 1); and
B) another 10 glass microcaps were filled with Briij 35 (3 % weight/ volume, Sigma prod.no. 228340050) in de-ionized water.

All 20 microcaps were thereafter emptied by contacting them to a paper tissue, and allowed to dry overnight at ambient temperature.

The next day the speed of inlet of microcaps without treatment were compared to the speed of inlet of microcaps treated with A) and B), respectively. See table 5.

The speed of inlet was determined by putting the end of each type of microcap into a venous (EDTA) blood sample and measuring the height of blood in each microcap after 3 seconds.

**Table 5**

| Type of microcap | Height of blood in microcap after 3 seconds* |
|---|---|
| Untreated | 30 mm |
| A) Reagent | 42 mm |
| B) Brij 35 | 43 mm |

| | |
|---|---|
| *Average of 10 runs. | |

### Conclusion:

The microcaps treated with the reagent of Example 1 were filled with blood with a speed in the same order as those filled with Brij 35.

## Claims

1. A reagent comprising
- a hemoglobin-stabilizing compound selected from the group consisting of imidazole, imidazole derivatives, and combinations thereof;
- at least two quaternary ammonium salts;
- 1,3-dimethylurea and/or salts thereof; and
- an organic buffer.

2. A reagent according to claim 1, wherein the concentration of the hemoglobin-stabilizing compound selected from the group consisting of imidazole, imidazole derivatives, and combinations thereof in the reagent is in the range from 10 mmol/L to 100 mmol/L, such as from 25 mmol/L to 75 mmol/L, from 40 mmol/L to 60 mmol/L, and preferably 50 mmol/L.

3. A reagent according to claim 1 or 2, where the hemoglobin-stabilizing compound is imidazole in a concentration of 50 mmol/L.

4. A reagent according to any of the preceding claims, wherein the at least two quaternary ammonium salts comprise a combination of dodecyltrimethylammonium chloride and hexadecyltrimethylammonium bromide.

5. A reagent according to any of the preceding claims, wherein the total concentration of the at least two quaternary ammonium salts in the reagent is in the range from 1.0 mmol/L to 10 mmol/L, such as from 3.0 mmol/L to 9.0 mmol/L, from 4.5 mmol/L to 8.5 mmol/L, and preferably 6.5 mmol/L.

6. A reagent according to any of the preceding claims, wherein the at least two quaternary ammonium salts is a combination of dodecyltrimethylammonium chloride and hexadecyltrimethylammonium bromide in concentrations of 5.6 mmol/L and 0.9 mmol/L, respectively.

7. A reagent according to any of the preceding claims, wherein the concentration of 1,3-dimethylurea and/or salts thereof in the reagent is in the range from 10 mmol/L to 25 mmol/L, such as from 12 mmol/L to 23 mmol/L, from 14 mmol/L to 20 mmol/L, from 16 mmol/L to 18 mmol/L, and preferably 17 mmol/L.

8. A reagent according to any of the preceding claims, wherein the 1,3-dimethylurea and/or salts thereof is 1,3-dimethylurea in a concentration of 17 mmol/L.

9. A reagent according to any of the preceding claims, wherein the reagent further comprises procaine, procaine hydrochloride or other salts thereof.

10. A reagent according to claim 9, wherein the concentration of procaine hydrochloride in the reagent is in the range from 0.2 mmol/L to 1.6 mmol/L, such as from 0.4 mmol/L to 1.4 mmol/L, from 0.6 mmol/L to 1.2 mmol/L, from 0.8 mmol/L to 1.0 mmol/L, and preferably 0.9 mmol/L.

11. A reagent according to any of the preceding claims, wherein the organic buffer is a compound such as ADA (N-2-(acetamido)iminodiacetic acid), HEPES (2-(4(2-hydroxyethyl)-1-piperazine)ethanesulphonic acid), MOPS (3-(N-morpholino)propane sulphonic acid), PIPES (piperazine-N,N'-bis(2-ethane sulphonic acid) or combinations thereof, with a concentration in the range from 2 mmol/L to 8 mmol/L, such as from 4 mmol/L to 6 mmol/L, from 5 mmol/L to 5.6 mmol/L, and preferably 5.3 mmol/L.

12. A reagent according to any of the preceding claims, wherein the organic buffer is ADA (N-2-(acetamido)iminodiacetic acid) with a concentration in the range from 2 mmol/L to 8 mmol/L, such as from 4 mmol/L to 6 mmol/L, from 5.0 mmol/L to 5.6 mmol/L, and preferably 5.3 mmol/L.

13. A reagent according to any of the preceding claims, wherein the organic buffer is ADA (N-2-(acetamido)iminodiacetic acid) with a concentration of 5.3 mmol/L.

14. A reagent according to any of the preceding claims, wherein the osmolality of the reagent is adjusted with at least one inorganic salt, such as sodium chloride, and/or sodium sulphate.

15. A reagent according to any of the preceding claims, wherein the osmolality of the reagent is adjusted with sodium chloride and sodium sulphate.

16. A reagent according to any of claims 14 and 15, wherein the osmolality of the reagent is in the range from 200 mosmol/L to 400 mosmol/L, such as from 250 mosmol/L to 380 mosmol/L, from 300 mosmol/L to 350 mosmol/L, and preferably 330 mosmol/L

17. A reagent according to any of the preceding claims, wherein the pH of the reagent is adjusted with an acid, and the pH of the reagent is in the range from 6 to 8, such as from 6.5 to 7.5, and preferably 7.1.

18. A reagent according to any of the preceding claims, comprising imidazole in a concentration of 50 mmol/L; dodecyltrimethylammonium chloride in a concentration of 5.6 mmol/L; hexadecyltrimethylammonium bromide in a concentration of 0.9 mmol/L; 1,3-dimethylurea in a concentration of 17 mmol/L; ADA (N-2-(acetamido)iminodiacetic acid) in a concentration of 5.3 mmol/L; procaine hydrochloride in a concentration of 0.9 mmol/L; and de-ionized water;
wherein pH is adjusted to 7.1 with diluted sulfuric acid and the osmolality is adjusted with sodium chloride and sodium sulphate.

19. A coating comprising a reagent as defined in any of claims 1-18.

20. A method of determining the content of blood cells in a blood sample, the method comprising the steps of:
i) Mixing a blood sample with a reagent as defined in any of claims 1-18, and
ii) Analyzing the content of the blood sample by employment of the Coulter principle.

21. A method according to claim 20, wherein the thrombocyte content is determined by step ii).

22. A method according to any of claims 20 and 21, wherein the leukocyte content is determined by step ii).

23. A method according to any of claims 20-22, wherein one or more leukocyte subpopulations such as, e.g., the lymphocytes, monocytes and/or granulocytes are determined by step ii).

24. A method according to any of claims 20-23, wherein the method further comprises
iii) Analyzing the content of the blood sample by spectrophotometric quantification of the hemoglobin content.

25. A method according to any of claims 20-24, wherein the blood:reagent-ratio by volume is in the range from 1:10,000 to 1:200, such as from 1:1,000 to 1:300, from 1:600 to 1:400, from 1:550 to 1:450, and preferably about 1:500.

26. A method according to any of claims 20-25, wherein
step i) is performed in a cartridge suitable for use in an apparatus for enumeration of cells in a blood sample, in which cartridge the reagent is present in a holding chamber that is connected with a mixing chamber in which the reagent is mixed with the blood sample; and
step ii) is performed by subsequent passage of the blood sample comprising cells through an orifice positioned between the mixing chamber and a collection chamber, whereby the content of the blood is analyzed by employment of the Coulter principle by cell characterization means.

27. A method according to any of claims 24-26, wherein step iii) is performed in a volume metering chamber connected to the collection chamber and adapted to spectrophotometric quantification.

28. Use of a reagent as defined in any of claims 1-18 for determination of the content of thrombocytes in a blood sample.

29. Use according to claim 28 for simultaneous determination of the content of leukocytes in the same blood sample.

30. Use according to any of claims 28 and 29 for simultaneous determination of one or more leukocyte subpopulations in the same blood sample.

31. Use according to claim 30, wherein the leukocyte subpopulation includes lymphocytes, monocytes and/or granulocytes.

32. Use according to any of claims 27-29 for determination of the hemoglobin content in the same blood sample.

## Patentansprüche

1. Reagenz, umfassend
- eine hämoglobinstabilisierende Verbindung, die aus der aus Imidazol, Imidazolderivaten und Kombinationen davon bestehenden Gruppe ausgewählt ist;
- zumindest zwei quaternäre Ammoniumsalze;
- 1,3-Dimethylurea und/oder Salze davon; und
- einen organischen Puffer.

2. Reagenz nach Anspruch 1, wobei die Konzentration der hämoglobinstabilisierenden Verbindung, die aus der aus Imidazol, Imidazolderivaten und Kombinationen davon bestehenden Gruppe ausgewählt ist, im Reagenz im Bereich von 10 mmol/L bis 100 mmol/L wie etwa von 25 mmol/L bis 75 mmol/L, von 40 mmol/L bis 60 mmol/L und bevorzugt 50 mmol/L liegt.

3. Reagenz nach Anspruch 1 oder 2, wobei die hämoglobinstabilisierende Verbindung ein Imidazol in einer Konzentration von 50 mmol/L darstellt.

4. Reagenz nach irgendeinem der vorhergehenden Ansprüche, wobei die zumindest zwei quaternären Ammoniumsalze eine Kombination von Dodecyltrimethylammoniumchlorid und Hexadecyltrimethylammoniumbromid umfassen.

5. Reagenz nach irgendeinem der vorhergehenden Ansprüche, wobei die Gesamtkonzentration der zumindest zwei quaternären Ammoniumsalze im Reagenz im Bereich von 1,0 mmol/L bis 10 mmol/L wie etwa von 3,0 mmol/L bis 9,0 mmol/L, von 4,5 mmol/L bis 8,5 mmol/L und bevorzugt 6,5 mmol/L liegt.

6. Reagenz nach irgendeinem der vorhergehenden Ansprüche, wobei die zumindest zwei quaternären Ammoniumsalze eine Kombination von Dodecyltrimethylammoniumchlorid und Hexadecyltrimethylammoniumbromid in Konzentrationen von 5,6 mmol/L beziehungsweise 0,9 mmol/L darstellen.

7. Reagenz nach irgendeinem der vorhergehenden Ansprüche, wobei die Konzentration von 1,3-Dimethylurea und/oder Salzen davon im Reagenz im Bereich von 10 mmol/L bis 25 mmol/L wie etwa von 12 mmol/L bis 23 mmol/L, von 14 mmol/L bis 20 mmol/L, von 16 mmol/L bis 18 mmol/L und bevorzugt 17 mmol/L liegt.

8. Reagenz nach irgendeinem der vorhergehenden Ansprüche, wobei die 1,3-Dimethylurea und/oder Salze davon eine 1,3-Dimethylurea in einer Konzentration von 17 mmol/L darstellen.

9. Reagenz nach irgendeinem der vorhergehenden Ansprüche, wobei das Reagenz Procain, Procainhydrochlorid oder andere Salze davon umfasst.

10. Reagenz nach Anspruch 9, wobei die Konzentration von Procainhydrochlorid im Reagenz im Bereich von 0,2 mmol/L bis 1,6 mmol/L wie etwa von 0,4 mmol/L bis 1,4 mmol/L, von 0,6 mmol/L bis 1,2 mmol/L, von 0,8 mmol/L bis 1,0 mmol/L und bevorzugt 0,9 mmol/L liegt.

11. Reagenz nach irgendeinem der vorhergehenden Ansprüche, wobei der organische Puffer eine Verbindung wie etwa ADA (N-2- (Acetamid)iminodiessigsäure), HEPES (2-(4(2-Hydroxyethyl)-1-Piperazin)ethansulfonsäure), MOPS (3-(N-Morpholino)propansulfonsäure), PIPES (Piperazin-N,N'-bis(2-Ethansulfonsäure) oder Kombinationen davon mit einer Konzentration im Bereich von 2 mmol/L bis 8 mmol/L wie etwa von 4 mmol/L bis 6 mmol/L, von 5 mmol/L bis 5,6 mmol/L und bevorzugt 5,3 mmol/L darstellt.

12. Reagenz nach irgendeinem der vorhergehenden Ansprüche, wobei der organische Puffer eine ADA (N-2-(Acetamid)iminodiessigsäure) mit einer Konzentration im Bereich von 2 mmol/L bis 8 mmol/L wie etwa von 4 mmol/L bis 6 mmol/L, von 5,0 mmol/L bis 5,6 mmol/L und bevorzugt 5,3 mmol/L darstellt.

13. Reagenz nach irgendeinem der vorhergehenden Ansprüche, wobei der organische Puffer eine ADA (N-2-(Acetamid)iminodiessigsäure) mit einer Konzentration von 5,3 mmol/L darstellt.

14. Reagenz nach irgendeinem der vorhergehenden Ansprüche, wobei die Osmolalität des Reagenzes mit zumindest einem anorganischen Salz wie etwa Natriumchlorid und/oder Natriumsulfat justiert ist.

15. Reagenz nach irgendeinem der vorhergehenden Ansprüche, wobei die Osmolalität des Reagenzes mit Natriumchlorid und Natriumsulfat justiert ist.

16. Reagenz nach irgendeinem der Ansprüche 14 und 15, wobei die Osmolalität des Reagenzes im Bereich von 200 mosmol/L bis 400 mosmol/L wie etwa von 250 mosmol/L bis 380 mosmol/L, von 300 mosmol/L bis 350 mosmol/L und bevorzugt 330 mosmol/L liegt.

17. Reagenz nach irgendeinem der vorhergehenden Ansprüche, wobei das pH des Reagenzes mit einer Säure justiert ist, und das pH des Reagenzes im Bereich von 6 bis 8 wie etwa von 6,5 bis 7,5 und bevorzugt 7,1 liegt.

18. Reagenz nach irgendeinem der vorhergehenden Ansprüche, umfassend Imidazol in einer Konzentration von 50 mmol/L; Dodecyltrimethylammoniumchlorid in einer Konzentration von 5,6 mmol/L; Hexadecyltrimethylammoniumbromid in einer Konzentration von 0,9 mmol/L; 1,3-Dimethylurea in einer Konzentration von 17 mmol/L ADA (N-2-(Acetamid)iminodiessigsäure) in einer Konzentration von 5,3 mmol/L; Procainhydrochlorid in einer Konzentration von 0,9 mmol/L; und deionisiertes Wasser; wobei das pH auf 7,1 mit verdünnter Schwefelsäure justiert ist, und die Osmolalität mit Natriumchlorid und Natriumsulfat justiert ist.

19. Beschichtung, umfassend ein Reagenz nach irgendeinem der Ansprüche 1-18.

20. Verfahren zur Bestimmung des Inhalts von Blutzellen in einer Blutprobe, wobei das Verfahren die folgenden Schritte enthält:
i) Mischen einer Blutprobe mit einem Reagenz nach irgendeinem der Ansprüche 1-18 und
ii) Analysieren des Inhalts der Blutprobe durch Verwendung des Coulter-Prinzips.

21. Verfahren nach Anspruch 20, wobei der Thrombozyteninhalt durch Schritt ii) bestimmt wird.

22. Verfahren nach irgendeinem der Ansprüche 20 und 21, wobei der Leukozyteninhalt durch Schritt ii) bestimmt wird.

23. Verfahren nach irgendeinem der Ansprüche 20-22, wobei eine oder mehrere Leukozytensubpopulationen wie etwa z.B. die Lymphozyten, Monozyten und/oder Granulozyten durch Schritt ii) bestimmt werden.

24. Verfahren nach irgendeinem der Ansprüche 20-23, wobei das Verfahren außerdem
iii) das Analysieren des Inhalts der Blutprobe durch spektrophotometrische Quantifizierung des Hämoglobininhalts umfasst.

25. Verfahren nach irgendeinem der Ansprüche 20-24, wobei das Blut:Reagenz-Volumenverhältnis im Bereich von ungefähr 1:10.000 bis 1:200 wie etwa von 1:1.000 bis 1:300, von 1:600 bis 1:400, von 1:550 bis 1:450 und bevorzugt 1:500 liegt.

26. Verfahren nach irgendeinem der Ansprüche 20-25, wobei
Schritt i) in einer für die Verwendung in einem Apparat zur Auszählung von Zellen in einer Blutprobe geeigneten Patrone ausgeführt wird, in welcher Patrone das Reagenz in einer mit einer Mischungskammer verbundenen Haltekammer vorhanden ist, in welcher Mischungskammer das Reagenz mit der Blutprobe gemischt wird; und
Schritt ii) bei anschließender Passage der Zellen umfassenden Blutprobe durch eine zwischen der Mischungskammer und einer Auffangkammer angebrachte Öffnung ausgeführt wird, wobei der Inhalt des Blutes durch Verwendung des Coulter-Prinzips mittels Zellencharakterisierungsmittel analysiert wird.

27. Verfahren nach irgendeinem der Ansprüche 24-26, wobei Schritt iii) in einer mit der Auffangkammer verbundenen und zur spektrophotometrischen Quantifizierung eingerichteten Volumenmessungskammer ausgeführt wird.

28. Verwendung eines Reagenzes nach irgendeinem der Ansprüche 1-18 zur Bestimmung des Inhalts von Thrombozyten in einer Blutprobe.

29. Verwendung nach Anspruch 28 zur gleichzeitigen Bestimmung des Inhalts von Leukozyten in derselben Blutprobe.

30. Verwendung nach irgendeinem der Ansprüche 28 und 29 zur gleichzeitigen Bestimmung einer oder mehrerer Leukozytensubpopulationen in derselben Blutprobe.

31. Verwendung nach Anspruch 30, wobei die Leukozytensubpopulation Lymphozyten, Monozyten und/oder Granulozyten enthält.

32. Verwendung nach irgendeinem der Ansprüche 27-29 zur Bestimmung des Hämoglobininhalts in derselben Blutprobe.

## Revendications

1. Réactif comprenant
- un composé stabilisant l'hémoglobine choisi dans le groupe constitué par l'imidazole, les dérivés de l'imidazole et les associations de ceux-ci ;
- au moins deux sels d'ammonium quaternaire;
- de la 1,3-diméthylurée et/ou des sels de celle-ci ; et
- un tampon organique.

2. Réactif selon la revendication 1, la concentration du composé stabilisant l'hémoglobine choisi dans le groupe constitué par l'imidazole, les dérivés de l'imidazole et les associations de ceux-ci dans le réactif étant dans la plage de 10 mmol/l à 100 mmol/l, telle que de 25 mmol/l à 75 mmol/l, de 40 mmol/l à 60 mmol/l, et de préférence 50 mmol/l.

3. Réactif selon la revendication 1 ou 2, dans lequel le composé stabilisant l'hémoglobine est l'imidazole en une concentration de 50 mmol/l.

4. Réactif selon l'une quelconque des revendications précédentes, dans lequel les au moins deux sels d'ammonium quaternaire comprennent une association de chlorure de dodécyltriméthylammonium et de bromure d'hexadécyltriméthylammonium.

5. Réactif selon l'une quelconque des revendications précédentes, la concentration totale des au moins deux sels d'ammonium quaternaire dans le réactif étant dans la plage de 1,0 mmol/l à 10 mmol/l, telle que de 3,0 mmol/l à 9,0 mmol/l, de 4,5 mmol/l à 8,5 mmol/l, et de préférence 6,5 mmol/l.

6. Réactif selon l'une quelconque des revendications précédentes, dans lequel les au moins deux sels d'ammonium quaternaire sont une association de chlorure de dodécyltriméthylammonium et de bromure d'hexadécyltriméthylammonium en concentrations de 5,6 mmol/l et 0,9 mmol/l, respectivement.

7. Réactif selon l'une quelconque des revendications précédentes, la concentration de la 1,3-diméthylurée et/ou des sels de celle-ci dans le réactif étant dans la plage de 10 mmol/l à 25 mmol/l, telle que de 12 mmol/l à 23 mmol/l, de 14 mmol/l à 20 mmol/l, de 16 mmol/l à 18 mmol/l, et de préférence 17 mmol/l.

8. Réactif selon l'une quelconque des revendications précédentes, dans lequel la 1,3-diméthylurée et/ou les sels de celle-ci est la 1,3-diméthylurée en une concentration de 17 mmol/l.

9. Réactif selon l'une quelconque des revendications précédentes, le réactif comprenant en outre de la procaïne, du chlorhydrate de procaïne ou d'autres sels de celle-ci.

10. Réactif selon la revendication 9, la concentration du chlorhydrate de procaïne dans le réactif étant dans la plage de 0,2 mmol/l à 1,6 mmol/l, telle que de 0,4 mmol/l à 1,4 mmol/l, de 0,6 mmol/l à 1,2 mmol/l, de 0,8 mmol/l à 1,0 mmol/l, et de préférence 0,9 mmol/l.

11. Réactif selon l'une quelconque des revendications précédentes, dans lequel le tampon organique est un composé tel que l'ADA (acide N-2-(acétamido)iminodiacétique), l'HEPES (acide 2-(4-(2-hydroxyéthyl)-1-pipérazine)éthanesulfonique), le MOPS (acide 3-(N-morpholino)propanesulfonique), le PIPES (pipérazine-N,N'-bis(acide 2-éthanesulfonique) ou des associations de ceux-ci, avec une concentration dans la plage de 2 mmol/l à 8 mmol/l, telle que de 4 mmol/l à 6 mmol/l, de 5 mmol/l à 5,6 mmol/l, et de préférence 5,3 mmol/l.

12. Réactif selon l'une quelconque des revendications précédentes, dans lequel le tampon organique est l'ADA (acide N-2-(acétamido)iminodiacétique) avec une concentration dans la plage de 2 mmol/l à 8 mmol/l, telle que de 4 mmol/l à 6 mmol/l, de 5,0 mmol/l à 5,6 mmol/l, et de préférence 5,3 mmol/l.

13. Réactif selon l'une quelconque des revendications précédentes, dans lequel le tampon organique est l'ADA (acide N-2-(acétamido)iminodiacétique) avec une concentration de 5,3 mmol/l.

14. Réactif selon l'une quelconque des revendications précédentes, l'osmolalité du réactif étant ajustée avec au moins un sel inorganique, tel que le chlorure de sodium et/ou le sulfate de sodium.

15. Réactif selon l'une quelconque des revendications précédentes, l'osmolalité du réactif étant ajustée avec du chlorure de sodium et du sulfate de sodium.

16. Réactif selon l'une quelconque des revendications 14 et 15, l'osmolalité du réactif étant dans la plage de 200 mosmol/l à 400 mosmol/l, telle que de 250 mosmol/l à 380 mosmol/l, de 300 mosmol/l à 350 mosmol/l, et de préférence 330 mosmol/l.

17. Réactif selon l'une quelconque des revendications précédentes, le pH du réactif étant ajusté avec un acide et le pH du réactif étant dans la plage de 6 à 8, tel que de 6,5 à 7,5, et de préférence 7,1.

18. Réactif selon l'une quelconque des revendications précédentes, comprenant de l'imidazole en une concentration de 50 mmol/l; du chlorure de dodécyltriméthylammonium en une concentration de 5,6 mmol/l; du bromure d'hexadécyltriméthylammonium en une concentration de 0,9 mmol/l; de la 1,3-diméthylurée en une concentration de 17 mmol/l; de l'ADA (acide N-2-(acétamido)iminodiacétique) en une concentration de 5,3 mmol/l; du chlorhydrate de procaïne en une concentration de 0,9 mmol/l; et de l'eau déminéralisée ;
le pH étant ajusté à 7,1 avec de l'acide sulfurique dilué et l'osmolalité étant ajustée avec du chlorure de sodium et du sulfate de sodium.

19. Revêtement comprenant un réactif tel que défini dans l'une quelconque des revendications 1-18.

20. Procédé de détermination de la teneur de cellules sanguines dans un échantillon de sang, le procédé comprenant les étapes consistant à:
i) mélanger un échantillon de sang avec un réactif tel que défini dans l'une quelconque des revendications 1-18 et
ii) analyser le contenu de l'échantillon de sang à l'aide du principe Coulter.

21. Procédé selon la revendication 20, dans lequel la teneur en thrombocytes est déterminée par l'étape ii).

22. Procédé selon l'une quelconque des revendications 20 et 21, dans lequel la teneur en leucocytes est déterminée par l'étape ii).

23. Procédé selon l'une quelconque des revendications 20-22, dans lequel une ou plusieurs sous-populations de leucocytes telles que, par exemple, les lymphocytes, les monocytes et/ou les granulocytes, sont mesurées par l'étape ii).

24. Procédé selon l'une quelconque des revendications 20-23, le procédé comprenant en outre
iii) l'analyse du contenu de l'échantillon de sang par quantification spectrophotométrique de la teneur en hémoglobine.

25. Procédé selon l'une quelconque des revendications 20-24, dans lequel le rapport sang:réactif en volume est dans la plage de 1:10 000 à 1:200, tel que de 1:1 000 à 1:300, de 1:600 à 1:400, de 1:550 à 1:450, et de préférence environ 1:500.

26. Procédé selon l'une quelconque des revendications 20-25, dans lequel
l'étape i) est effectuée dans une cartouche convenant pour être utilisée dans un appareil pour la numération de cellules dans un échantillon de sang, dans laquelle cartouche le réactif est présent dans une chambre de stockage qui est reliée à une chambre de mélange dans laquelle le réactif est mélangé avec l'échantillon de sang ; et
l'étape ii) est effectuée par passage subséquent de l'échantillon de sang comprenant des cellules dans un orifice placé entre la chambre de mélange et une chambre de collecte, ce par quoi le contenu du sang est analysé à l'aide du principe Coulter par des moyens de caractérisation de cellules.

27. Procédé selon l'une quelconque des revendications 24-26, dans lequel l'étape iii) est effectuée dans une chambre volumétrique reliée à la chambre de collecte et adaptée à une quantification spectrophotométrique.

28. Utilisation d'un réactif tel que défini dans l'une quelconque des revendications 1-18 pour la détermination de la teneur en thrombocytes dans un échantillon de sang.

29. Utilisation selon la revendication 28 pour la détermination simultanée de la teneur en leucocytes dans le même échantillon de sang.

30. Utilisation selon l'une quelconque des revendications 28 et 29 pour la mesure simultanée d'une ou plusieurs sous-populations de leucocytes dans le même échantillon de sang.

31. Utilisation selon la revendication 30, la sous-population de leucocytes comprenant les lymphocytes, les monocytes et/ou les granulocytes.

32. Utilisation selon l'une quelconque des revendications 27-29 pour la détermination de la teneur en hémoglobine dans le même échantillon de sang.
